Europäisches Patentamt

European Patent Office

Office européen des brevets

(11) Publication number: **0 275 642 B1**

# EUROPEAN PATENT SPECIFICATION

(45) Date of publication of patent specification: **16.09.92**   (51) Int. Cl.⁵: **A61N  1/26**

(21) Application number: **87310403.8**

(22) Date of filing: **25.11.87**

(54) **Needle base for subcutaneously applying electric current for use in plastic surgery.**

(30) Priority: **20.01.87 JP 6395/87**

(43) Date of publication of application:
**27.07.88 Bulletin  88/30**

(45) Publication of the grant of the patent:
**16.09.92 Bulletin  92/38**

(84) Designated Contracting States:
**DE FR GB**

(56) References cited:
CH-A- 243 478       DE-A- 186 177
DE-A- 197 833       FR-A- 2 582 522
GB-A- 291 865       GB-A- 343 995
GB-A- 635 438       US-A- 3 994 300

(73) Proprietor: **Takase, Haruo**
**20-16, 3-chome, Shimoochiai Shinjuku-ku**
**Tokyo(JP)**

(72) Inventor: **Takase, Haruo**
**20-16, 3-chome, Shimoochiai Shinjuku-ku**
**Tokyo(JP)**

(74) Representative: **Ben-Nathan, Laurence Albert**
**et al**
**Urquhart-Dykes & Lord 91 Wimpole Street**
**London W1M 8AH(GB)**

## Description

This invention relates to a needle base for use in subcutaneously applying electric current in plastic surgery, and more particularly to a needle base which is implanted with a plurality of needles at predetermined intervals on one side thereof for passing electric current and which connects all the needles conductively on the bottoms thereof so as to enable simultaneous application of predetermined electric current to these needles.

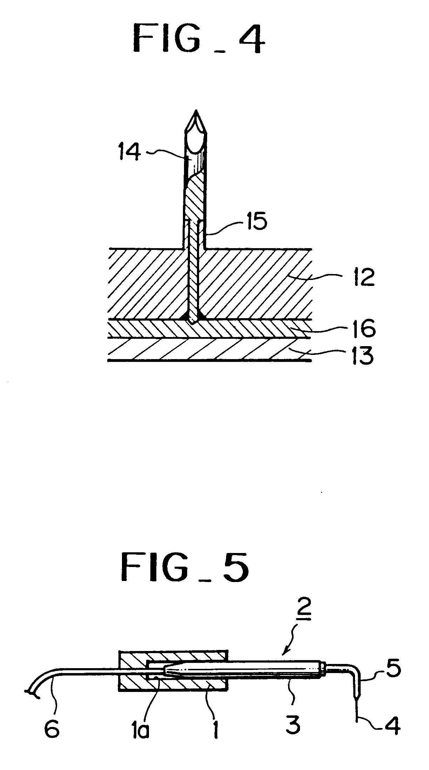

In the prior art, the needles shown in Fig. 5 have been used to apply subcutaneously electric current for cauterization and/or coagulation of hair follicles, subcutaneous adipose tissues or sebaceous glands in order to remove the hair in plastic surgeries. Such a needle comprises a jig 1 made of synthetic resin and an attachment needle member 2 which is to be inserted into a hole 1a of the jig 1. The attachment needle member 2 comprises an electrode 3, a needle 4 which is bent substantially at a right angle from the end of the electrode, and a non-conductive member 5 made of synthetic resin or other material which is placed over said needle 4 at the exposed part thereof extending from the electrode 3 except for the tip end which is to be inserted into the skin. A wire 6 is connected to the jig 1 at the rear end thereof so as to reach the electrode 3. In practice, the jig 1 is held with fingers to position the end of a needle 4 in a pore on the skin at a surgery site, and pushed down into the skin to a predetermined depth until the end of said non-conductive member 5 abuts on the skin. A predetermined electric current is then supplied to perform such surgery operations as cauterization, coagulation, etc. of hair follicles, subcutaneous adipose tissues or sebaceous glands.

When used for plastic surgery, it is necessary to prick the skin with a plurality of sets of jigs 1 and the attachment needle member 2 in a pattern parallel to each other but with an interval of generally 3 - 5 mm, and to supply a predetermined electric current to all of the needles respectively. The operation therefore needs considerable skill and experience as well as cumbersome manipulations. Moreover, the prior art needles tend to apply electric current on the upper surface cuticles in addition to the surgery site penetrated with the needles to thereby inconveniently affect and damage the skin surface by cauterization.

US-A-3,994,300 discloses an epilating device having an array of electrically connected electrodes for use with a cooperating electrically conductive grid placed on an area of skin to provide electrical discharges between the electrodes and ends of previously shaven hair shafts protruding through the grid.

GB-A-291,865 discloses an electrode base in the form of a hairbrush but adapted for electric massage or therapeutic treatment when placed in contact with a surface area of skin. A similar device is disclosed in DE-A-186,177.

CH-A-243 478 discloses an epilation device comprising a plurality of needles for insertion into the skin, the needles being disposed in a common holder. The needles are connected to a common electrical supply.

An object of this invention is to provide an improved needle base for passing electric current subcutaneously which overcomes or at least minimizes the above-mentioned problems of the prior art described above, and is particularly suitable for treatment of curved or resilient treatment areas.

The invention provides a needle base according to Claim 1.

A needle base according to the invention enables a plurality of needles to be inserted under the skin simultaneously and an electric current supplied to all needles simultaneously thereby to remarkably shorten the operating time as well as to simplify the surgery.

The invention provides a needle base which can treat precisely a specific subcutaneous site to apply an electric current thereto by inserting a plurality of needles which are arranged at predetermined spaced intervals, e.g., in a parallel relationship, to thereby simplify the surgery procedure.

The invention also provides a needle base for subcutaneously supplying electric current in plastic surgery which can insert a plurality of needles to a uniform subcutaneous depth even at resilient locations such as the abdomen.

An embodiment of the invention will now be described by way of example and with reference to the accompanying drawings, in which:-

Fig. 1 is a perspective view of a needle base for subcutaneous electric current supply for use in plastic surgery which is not in accordance with the present invention;

Fig. 2 is a cross sectional view on an enlarged scale of the needle base of Fig. 1;

Fig. 3 is a partial cross-section of a needle base embodying the invention,

Fig. 4 is an enlarged view of the portion of the needle base encircled at A in Fig. 2 as used in Fig. 3; and,

Fig. 5 is a cross sectional view illustrating a part of a prior art needle for subcutaneously supplying electric current.

This invention relates to a needle base for subcutaneously supplying electric current which can be easily used in various types of plastic surgery without requiring surgical expertise, yet effectively for such purposes as cauterization of hair follicles and/or cauterization or coagulation of sebaceous glands to remove hair etc.

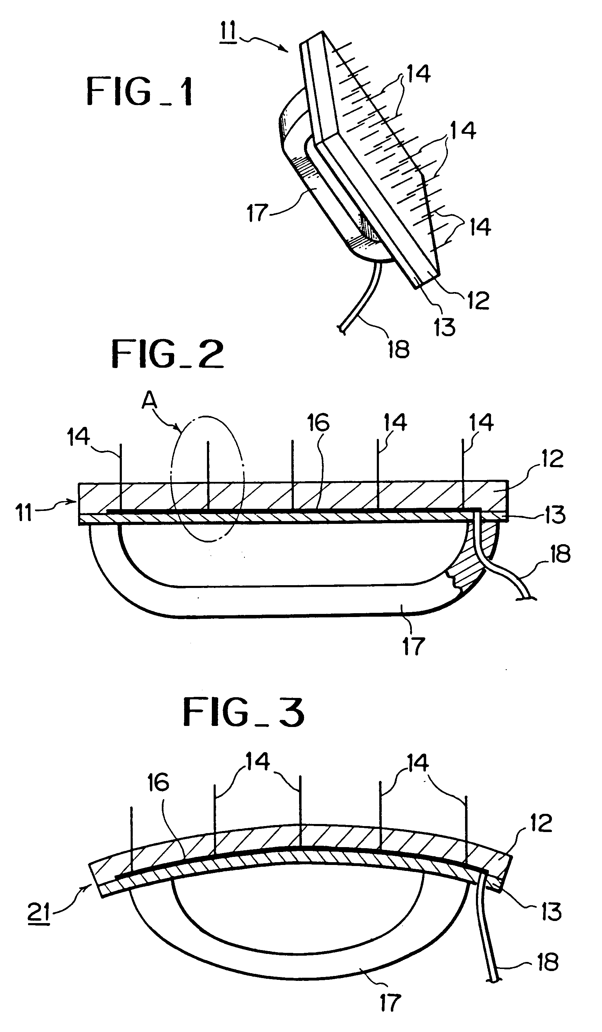

The needle base illustrated in Figs. 1, 2 and 4, which is not in accordance with the invention, is shown because it includes constructional features of a needle base in accordance with the invention, as illustrated in Fig. 3.

Referring to Figs. 1, 2 and 4, numeral 11 denotes an insulated substrate, and numerals 14 denotes a plurality of needles for subcutaneously supplying electric current. Base portions of the needles are embedded in one side of the substrate 11 with a predetermined interval therebetween, e.g. 5 mm.

The substrate 11 comprises a laminated structure which consists of a plate 12 and a cover plate 13. As shown in Fig. 4, the needles 14 subcutaneously supplying electric current are provided on the plate 12 with their base portions embedded in, and their tip ends projecting from, one surface of the plate 12, each needle being partially covered with a non-conductive member 15 made of plastics or other insulating materials in a manner such that the covered portion and the exposed portion of each needle have substantially the same diameter. The other surface of the plate 12 is provided with a printed circuit of conductive members 16 which are printed on the plate 12 in a grid pattern aligned with the pattern of the needles 14 to be electrically connected thereby. The cover plate 13, which is made of an insulating material, such as plastics, is integrally laminated over the whole surface of the other surface of the plate 12 and secured by an appropriate adhesive agent. Numeral 17 denotes a handle attached to the substrate 11, and numeral 18 denotes an electric core which is connected to a part of the pattern of conductive members 16.

Fig. 3 shows an embodiment of this invention. The structure of this embodiment is similar to that of the needle base illustrated in Figs. 1, 2 and 4 except that the substrate 21 implanted with the needles 14 is convex to have its highest point at the center thereof. The substrate 21 therefore comprises a convex non-conductive plate 12 and a convex cover plate 13.

In plastic surgery, after administering local anesthetic on the specified site and disinfecting the site with alcohol etc., the handle 17 is gripped allowing the needle base to be tightly pressed against that site so its surface implanted with the needles 14 is applied to the skin with the needles 14 being pushed into the skin.

A predetermined electric current is then supplied to the conductive member 16 inside the substrate 11 via the electric core 18 to apply an electric current to the skin at a predetermined depth at an amount generally used for coagulation to achieve coagulation of sebaceous glands, coagulation or cauterization of hair follicles or other plastic surgery.

As described above, a needle base according to the invention allows the subcutaneous insertion of a large number of needles simultaneously at an appropriate interval therebetween to a predetermined depth in the skin in precise positional relation without requiring any considerable skill or complex manipulations which were heretofore needed to achieve the same effect.

A needle base according to the invention can further save time needed for surgery to a remarkable extent as the base can supply electric current to all the needles simultaneously unlike prior art devices which feed electric current separately to individual needles. If the substrate is convex in accordance with the present invention to increase the height of the needles at the centre thereof as shown in Fig. 3, the needles implanted at or near the centre can be inserted to a relatively deeper depth in the skin to thereby enable insertion of the needles evenly into the skin if the site is curved.

As the lower portion near the base portion of a needle is coated with a non-conductive member 15 to a predetermined height, the skin will not be affected by cauterization at all except at the required sites and at the predetermined depth. A needle base according to the invention is advantageous, moreover, in that coagulation and cauterization can be performed at a large number of locations simultaneously.

## Claims

1. A needle base for subcutaneously supplying electric current in plastic surgery, comprising a plurality of needles (14) which are capable of being inserted into an area of skin to a predetermined depth in the skin for the subcutaneous supply of electric current and are mounted on and project a uniform distance from the surface of a non-conductive plate (12) at spaced intervals therebetween with base portions of the needles implanted in the the plate (12), the base portions of said needles (14) being conductively connected so as to enable simultaneous application of predetermined electric current thereto, said needles comprising a shank portion and a pointed leading end to facilitate insertion into the skin, with the lower part of said shank portion, near the bottom thereof, being covered with a non-conductive member (15), and the covered and exposed portions of each needle shank having substantially the same external diameter, wherein said non-conductive plate (12) is convex to have a protruding centre portion.

2. A needle base as claimed in Claim 1 wherein a non-conductive substrate (21) comprises the plate (12) and a cover (13), said base portions of the needles (14) being embedded in the plate (12) with the needles projecting from one side surface of the plate (12) and with said base portions of the needles (14) being conductively connected to each other on the opposite side of the plate (12), and said cover plate (13) being laminated on said opposite side surface of the plate (12).

3. A needle base as claimed in Claim 1 or Claim 2 wherein a handle (17) is integrally provided on the side of the plate (12) opposite to that from which the needles project.

**Patentansprüche**

1. Nadelhalter zum subkutanen Einführen eines elektrischen Stromes bei plastischer Chirurgie, bestehend aus einer Mehrzahl von Nadeln (14), welche in einem bestimmten Hautbereich bis zu einer vorgeschriebenen Hauttiefe zur subkutanen Einleitung eines elektrischen Stromes vorgesehen sind, wobei diese Nadeln jeweils unter Einhaltung eines gleichmäßigen Abstandes sich von der Oberfläche einer nichtleitenden Hauptplatte (12) erstrecken, wobei die in der Hauptplatte (12) implantierten Basisbereiche der Nadeln (14) elektrisch derart miteinander verbunden sind, daß gleichzeitig elektrische Ströme vorgegebener Größe durch die Nadeln (14) geleitet werden, wobei die Nadeln jeweils einen Schaftbereich und einen das Einführen in die Haut erleichternde Spitze aufweisen und wobei der untere Teil des Schaftbereiches mit einer nichtleitenden Schicht (15) abgedeckt ist und die abgedeckten und freiliegenden Bereiche der Nadelschäfte jeweils im wesentlichen denselben Außendurchmesser besitzen,
dadurch **gekennzeichnet,**
daß die nichtleitende Hauptplatte (12) konvex ausgebildet ist und einen vorspringenden mittleren Bereich besitzt.

2. Nadelhalter nach Anspruch 1, dadurch gekennzeichnet, daß das nichtleitende Substrat (21) eine Hauptplatte (12) und eine Abdeckplatte (13) aufweist, wobei die Basisbereiche der Nadeln (14) in der Hauptplatte (12) derart eingebettet sind, daß sich die Nadeln von der einen Oberfläche der Hauptplatte (12) weg erstrekken, während die Basisbereiche der Nadeln (14) auf der gegenüberliegenden Seite der Hauptplatte (12) elektrisch miteinander verbunden sind, und wobei auf der gegenüberliegenden Siete der Hauptplatte (12) eine Abdeckplatte (13) aufgebracht ist.

3. Nadelhalter nach Anspruch 1 oder 2, dadurch gekennzeichnet, daß die Hauptplatte (12) gegenüberliegend zu der Seite, an welcher sich die Nadeln erstrecken, mit einem Handgriff (17) versehen ist.

**Revendications**

1. Support d'aiguilles pour l'application souscutanée de courant électrique en chirurgie plastique, comprenant une pluralité d'aiguilles (14), qui sont susceptibles d'être insérées dans une zone cutanée, jusqu'à une profondeur prédéterminée de la peau, pour l'application souscutanée de courant électrique et sont montées sur, et font saillie d'une distance uniforme de, la surface d'une plaque non-conductrice (12), en formant des intervalles espacés entre elles, les parties de base des aiguilles (14) étant implantées dans la plaque (12) et étant reliées de façon conductrice, de manière à leur permettre une application simultanée de courant électrique prédéterminé, lesdites aiguilles comprenant une partie de tige et une extrémité avant pointue pour faciliter l'insertion dans la peau, la partie inférieure de ladite partie de tige, près de son fond, étant recouverte par un élément non-conducteur (15), et les parties recouvertes et exposées de chaque tige d'aiguille présentant sensiblement le même diamètre extérieur, dans lequel ladite plaque non-conductrice (12) est convexe pour présenter une partie centrale saillante.

2. Support d'aiguilles selon la revendication 1, dans lequel un substrat non-conducteur (21) comprend la plaque (12) et un recouvrement (13), lesdites parties de base des aiguilles (14) étant noyées dans la plaque (12), les aiguilles faisant saillie d'une surface latérale de la plaque (12) et lesdites parties de base des aiguilles (14) étant reliées entre elles de manière conductrice, sur le côté opposé de la plaque (12), et ladite plaque de recouvrement (13) étant stratifiée sur ladite surface latérale opposée de la plaque (12).

3. Support d'aiguilles selon la revendication 1 ou 2, dans lequel une poignée (17) est prévue d'un seul tenant sur le côté de la plaque (12), opposé à celui depuis lequel les aiguilles font saillie.

FIG_1

FIG_2

FIG_3

# FIG _ 4

# FIG _ 5